# EUROPEAN PATENT APPLICATION

(11) **EP 4 722 214 A1**
(43) Date of publication of application: **08.04.2026**
(21) Application number: 24814739.9
(22) Date of filing: 30.05.2024
(51) Int. Cl.: C07D 471/04, A61K 31/4375

(54) **METHOD OF PREPARING FINERENONE, INTERMEDIATE COMPOUND OF FINERENONE, AND METHOD OF PREPARING THE INTERMEDIATE COMPOUND**

(30) Priority: 31.05.2023 KR 20230069751
(71) Applicant: JW Pharmaceutical Corporation, Gyeonggi-do 13840 (KR)
(72) Inventor: LEE, Youngju, Seongnam-si Gyeonggi-do 13639 (KR); PARK, Jieun, Cheonan-si Chungcheongnam-do 31207 (KR); YOON, Dooha, Seongnam-si Gyeonggi-do 13562 (KR); CHIN, Seiho, Hwaseong-si Gyeonggi-do 18297 (KR)
(74) Representative: HGF
(86) International application number: PCT/IB2024/055237
(87) International publication number: WO 2024/246787

(57) **Abstract**

The present invention relates to a method for preparing finerenone, an intermediate compound of finerenone, and a method for preparing the same. Finerenone is prepared by using an intermediate compound represented by formula 2a or an intermediate compound represented by formula 3. 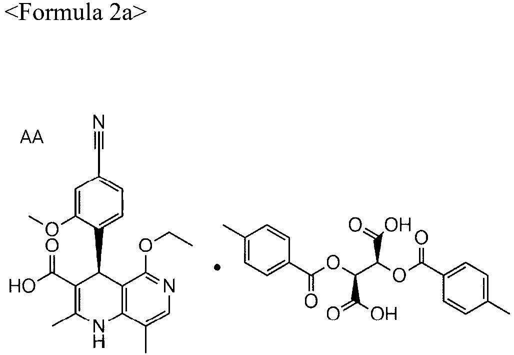 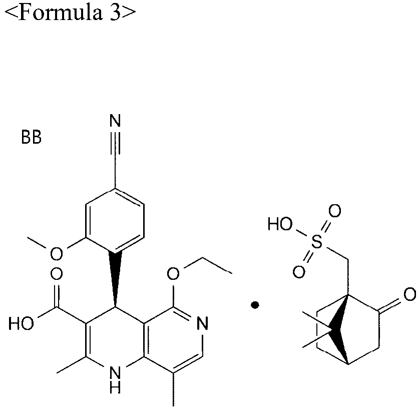 AA Chemical formula 2a
BB Chemical formula 3

## Description

### TECHNICAL FIELD

The present invention relates to a method for preparing finerenone, an intermediate compound of finerenone, and a method for preparing the same.

### BACKGROUND

Finerenone is a nonsteroidal selective mineralocorticoid receptor antagonist (MRA) having a mechanism which selectively acts on mineralocorticoid receptors to block effects harmful to the kidneys and heart.

A chemical name of finerenone is (4S)-4-(4-cyano-2-methoxyphenyl)-5-ethoxy-2,8-dimethyl-1,4-dihydro-1,6-naphthyridin-3-carboxamide, and is represented by formula I below.

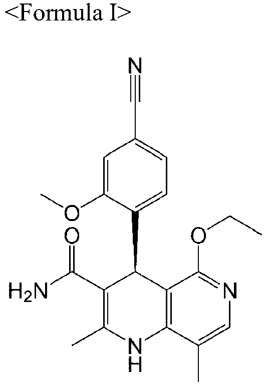

Finerenone is a drug having optical activity, and thus essentially requires a process of optically separating a specific form during a preparation process. In particular, there is a need for a method for preparing finerenone having a stable and excellent yield and optical purity while reducing costs, the number of processes, process times, and the like, in mass production.

### [Related Art Reference]

### [Patent Documents]

(Patent Document 1) Korean Registered Patent No. 10-1614164

### DETAILED DESCRIPTION OF THE INVENTION

### TECHNICAL PROBLEM

One object of the present invention is to provide a method for preparing finerenone with a consistently excellent yield and optical purity.

One object of the present invention is to provide an intermediate compound for preparing finerenone and a method for preparing the same.

### TECHNICAL SOLUTION

A method for preparing finerenone for one object of the present invention includes:
a step of preparing compound 2a represented by formula 2a or compound 3 represented by formula 3 by using compound 1 represented by formula 1; and
a step of preparing finerenone represented by formula I from compound 2a or 3.

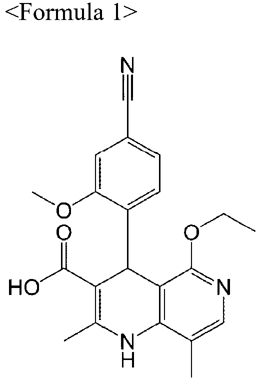

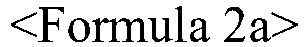

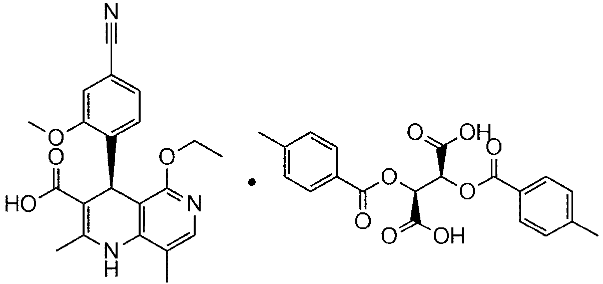

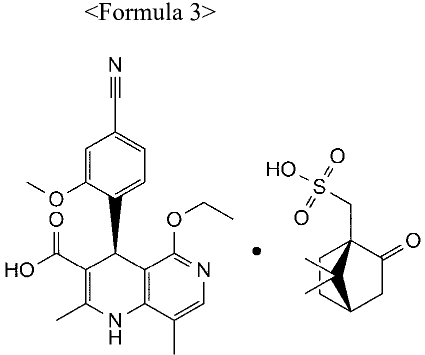

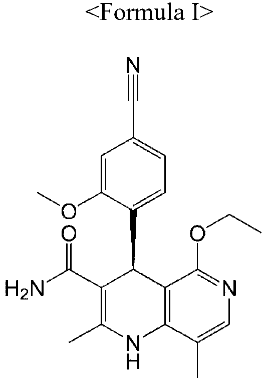

In the present invention, compound 1 represented by formula 1means a racemic mixture including compound 1a represented by formula 1a and compound 1b represented by formula 1b.

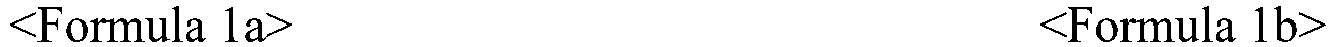

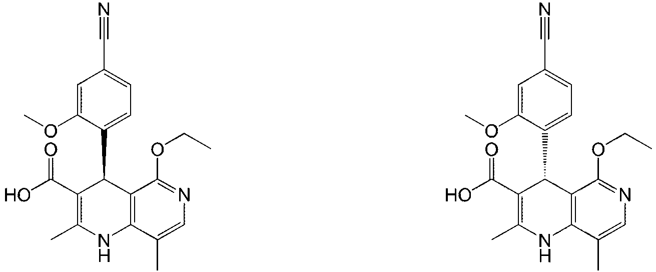

In one embodiment, the step of preparing compound 2a or 3 may include a step of reacting compound 1 and di-p-toluoyl-D-tartaric acid or (-)-camphor-10-sulfonic acid.

In the present invention, di-p-toluoyl-D-tartaric acid means a compound which is (2S,3S)-2,3-bis((4-methylbenzoyl)oxy)succinic acid under the IUPAC name.

In the present invention, (-)-camphor-10-sulfonic acid means a compound which is ((1R,4S)-7,7-dimethyl-2-oxobicyclo[2.2.1]heptan-1-yl)methanesulfonic acid under the IUPAC name.

In one embodiment, compound 1 and di-p-toluoyl-D-tartaric acid or (-)-camphor-10-sulfonic acid may be reacted in a presence of acetonitrile (ACN).

In one embodiment, the step of preparing compound 2a or 3 may include a step of reacting compound 1 and di-p-toluoyl-D-tartaric acid or (-)-camphor-10-sulfonic acid at 15 to 90°C.

In one embodiment, the step of preparing compound 2a or 3 may include a step of reacting compound 1 and di-p-toluoyl-D-tartaric acid under a condition of an organic solvent. In this case, the organic solvent may be methanol, ethanol, THF, or acetonitrile, which may be used alone or in combination of two or more thereof.

In one embodiment, when ethanol is used as an organic solvent, ethanol may be used alone or together with water and/or acetone. For example, when ethanol is used as the organic solvent, 100% ethanol, a mixed solvent in which a volume ratio of ethanol and water is 70:30, or a mixed solvent in which a volume ratio of ethanol and acetone is 50:50 to 70:30 may be used as the organic solvent. In one embodiment, compound 1 and di-p-toluoyl-D-tartaric acid may be reacted under a condition of the organic solvent including ethanol, water, and acetone all.

In one embodiment, when methanol is used as an organic solvent, methanol may be used alone, or methanol may be used together with ethyl acetate (EA), acetone, methyl acetate (MA), acetonitrile, dichloromethane (DCM), toluene, and methyl tertiary butyl ether (MTBE), or methanol may be used together with THF or acetone.

In one embodiment, the step of preparing compound 2a by reacting compound 1 and dip-toluoyl-D-tartaric acid under a condition of an organic solvent may be represented by reaction formula 1 below.

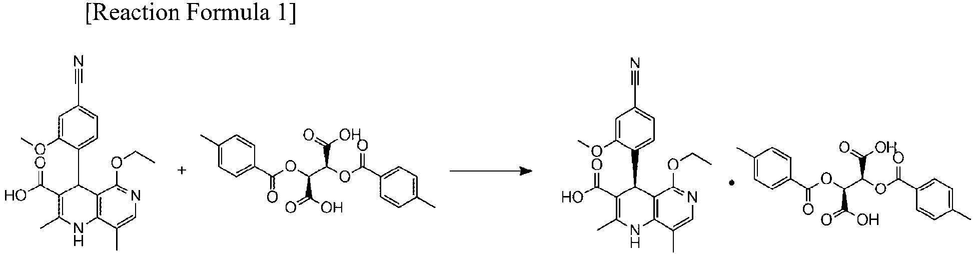

In one embodiment, compound 1 and di-p-toluoyl-D-tartaric acid may be mixed at an equivalent ratio of 1:0.5 to 1:3. For example, compound 1 and di-p-toluoyl-D-tartaric acid may be mixed at an equivalent ratio of 1:0.5 to 1:0.6.

In one embodiment, compound 1 and di-p-toluoyl-D-tartaric acid may be mixed at an equivalent ratio of 1:0.4 to 1:0.6. For example, compound 1 and di-p-toluoyl-D-tartaric acid may be mixed at an equivalent ratio of 1:0.55.

In one embodiment, the step of preparing compound 2a may include:
a step of mixing compound 1 and di-p-toluoyl-D-tartaric acid with acetonitrile;
a step of reacting and refluxing the resulting mixture at 70 to 90°C;
a step of cooling to a temperature lower than the reaction temperature; and
a step of obtaining compound 2a as a solid by filtering and washing.

In one embodiment, the step of cooling may include: a step of primarily cooling to 40 to 60°C; and a step of secondarily cooling to 20 to 30°C.

In one embodiment, the step of reacting and refluxing may be performed at 80°C. The primary cooling may be performed at 50°C, and the secondary cooling may be performed at 20 to 25°C.

In one embodiment, in the step of reacting compound 1 and di-p-toluoyl-D-tartaric acid under a condition of acetonitrile, acetonitrile may be mixed in a volume of 20 to 100 times the weight of compound 1.

In one embodiment, the step of preparing compound 2a may include:
a step of reacting compound 1 and di-p-toluoyl-D-tartaric acid under a condition of an organic solvent including either methanol or ethanol at 15 to 30°C; and
a step of obtaining compound 2a as a solid by filtering and washing. In this case, the organic solvent condition may be used as methanol alone, ethanol alone, a mixture of ethanol and water, a mixture of ethanol and acetone, a mixture of methanol and THF, or a mixture of ethanol, water and acetone. Solid compound 2a may be easily obtained through a simple process under a mild condition.

In one embodiment, when the organic solvent includes ethanol, in the mixing of compound 1 and di-p-toluoyl-D-tartaric acid with ethanol, ethanol may be mixed in a volume of 150 to 250 times the weight of compound 1. For example, ethanol may be mixed in a volume of 200 times the weight of compound 1.

In one embodiment, when the organic solvent includes methanol and THF, in the mixing of compound 1 and di-p-toluoyl-D-tartaric acid with methanol and THF, methanol may be mixed in a volume of 25 times the weight of compound 1, and THF may be mixed in a volume of 5 to 35 times the weight of compound 1.

In one embodiment, the step of preparing compound 2a may include:
a step of mixing compound 1 and di-p-toluoyl-D-tartaric acid with methanol and THF;
a step of reacting and stirring the resulting mixture at 20 to 22°C; and
a step of obtaining compound 2a as a solid by filtering, washing and drying under vacuum.

In one embodiment, in the step of reacting compound 1 and di-p-toluoyl-D-tartaric acid under a condition of methanol and THF, methanol may be mixed in a volume of 25 times the weight of compound 1, and THF may be mixed in a volume of 15 times the weight of compound 1.

In one embodiment, the step of preparing compound 3 may include a step of reacting compound 1 and (-)-camphor-10-sulfonic acid under a condition of an organic solvent. In this case, the organic solvent may be acetonitrile.

In one embodiment, in the mixing of compound 1 and (-)-camphor-10-sulfonic acid with acetonitrile, acetonitrile may be mixed in a volume of 10 times the weight of compound 1.

In one embodiment, the step of preparing compound 3 by reacting compound 1 and (-)-camphor-10-sulfonic acid under a condition of an organic solvent may be represented by reaction formula 2 below.

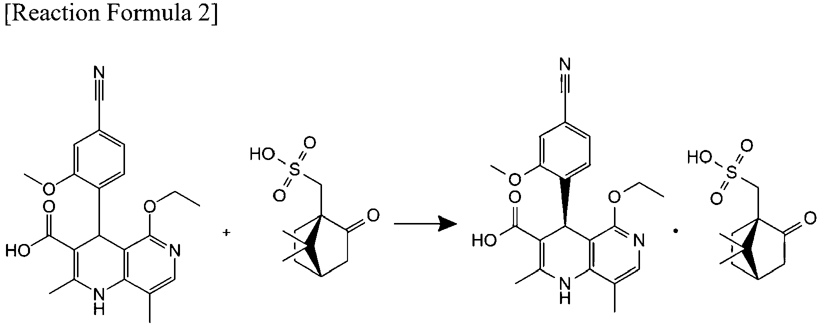

In one embodiment, compound 1 and (-)-camphor-10-sulfonic acid may be mixed at an equivalent ratio of 1:1 to 1:1.5. For example, compound 1 and (-)-camphor-10-sulfonic acid may be mixed at an equivalent ratio of 1:1.2.

In one embodiment, the step of preparing compound 3 may include:
a step of mixing compound 1 and (-)-camphor-10-sulfonic acid with acetonitrile;
a step of reacting and refluxing the resulting mixture at 70 to 90°C;
a step of cooling to a temperature lower than the reaction temperature; and
a step of obtaining compound 3 as a solid by filtering and washing. The step of cooling may be performed at 20 to 30°C.

In one embodiment, the step of preparing finerenone may include obtaining compound 1a using compound 2a or 3. The step of obtaining compound 1a using compound 2a or 3 may be a desalting process.

In one embodiment, the step of obtaining compound 1a with compound 2a or 3 may be performed by adding disodium phosphate (Na₂HPO₄) under a condition of an aqueous alcohol solution to carry out a reaction. In this case, the aqueous alcohol solution may be an aqueous ethanol solution.

In one embodiment, in the step of separating of compound 1a using compound 2a or 3, compound 1a may be obtained as a solid.

In one embodiment, the step of preparing finerenone may include a step of obtaining finerenone by using compound 1a, obtained by performing a desalting process on compound 2a or 3, as a reactant of amide coupling, in which the step of obtaining finerenone from compound 1a may be performed by reacting 1-ethyl-3-(3-dimethylaminopropyl)carbodiimide (EDC), 4-dimethylaminopyridine (DMAP), and ammonium hydroxide (NH₄OH) with compound 1a in tetrahydrofuran (THF). In this case, a reaction temperature may be 70 to 80°C.

In one embodiment, the preparing of finerenone using compound 2a or 3 may be represented by reaction formula 3 or 4 below.

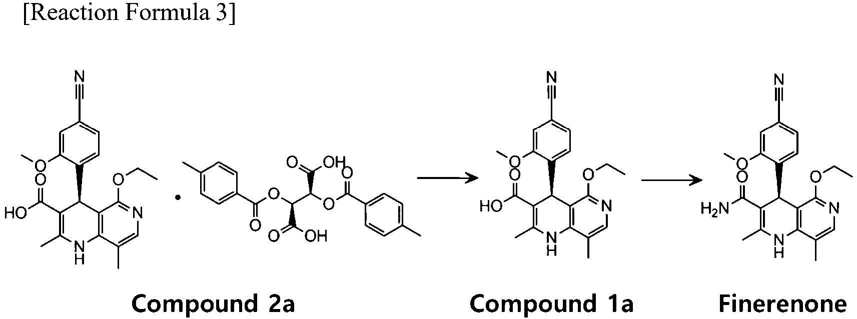

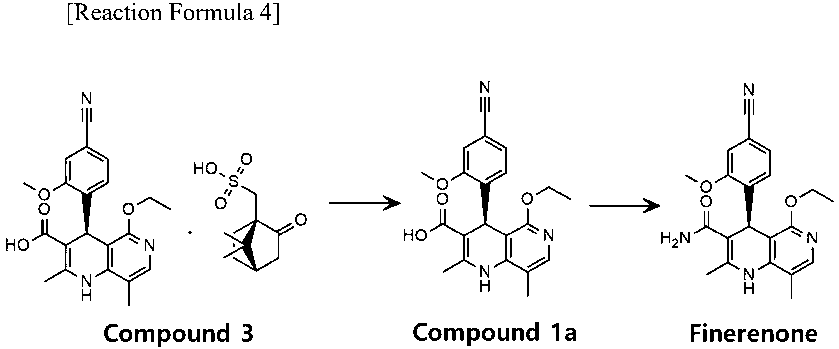

In one embodiment, the step of preparing finerenone may further include a step of obtaining compound 2a or 3 by using compound 1a obtained after the step of obtaining compound 1a using compound 2a or 3, and the step of obtaining compound 2a or 3 may be a salting process, which may be included to increase an optical purity of finerenone prepared.

In one embodiment, the step of obtaining compound 2a using compound 1a may include a step of reacting compound 1a and di-p-toluoyl-D-tartaric acid under a condition of an organic solvent, and a reaction temperature may be 20 to 22°C. For example, compound 1a and di-p-toluoyl-D-tartaric acid may be mixed at an equivalent ratio of 1:0.1 to 1:0.9.

In one embodiment, in the step of reacting of compound 1a and di-p-toluoyl-D-tartaric acid in an organic solvent, when methanol is used as the organic solvent, methanol may be used alone or together with THF. For example, when methanol is used alone, methanol may be mixed in a volume of 25 or 50 times the weight of compound 1a, and when methanol is used together with THF, methanol and THF may be mixed in volumes of 8 to 33 times and 2 to 20 times the weight of compound 1a, respectively. For example, compound 1a and di-p-toluoyl-D-tartaric acid may be reacted under a condition of an organic solvent including both methanol and THF, in which compound 1a and di-p-toluoyl-D-tartaric acid may be mixed at an equivalent ratio of 1:0.8, and methanol and THF may be mixed in volumes of 15 and 5 times the weight of compound 1a, respectively.

In one embodiment, the step of preparing finerenone may include a step of obtaining finerenone by using compound 2a or 3, obtained by performing a salting process on compound 1a, as a reactant of amide coupling without a separated desalting process.

In one embodiment, the step of obtaining finerenone by using compound 2a or 3 as a reactant of amide coupling may be performed by reacting 1-ethyl-3-(3-dimethylaminopropyl)carbodiimide (EDC), 4-dimethylaminopyridine (DMAP) and ammonium hydroxide (NH₄OH) with compound 2a or 3 in ethyl acetate (EA). In this case, a reaction temperature may be 70 to 80°C.

In one embodiment, the step of preparing finerenone using compound 2a or 3 may be represented by reaction formula 5 or 6 below.

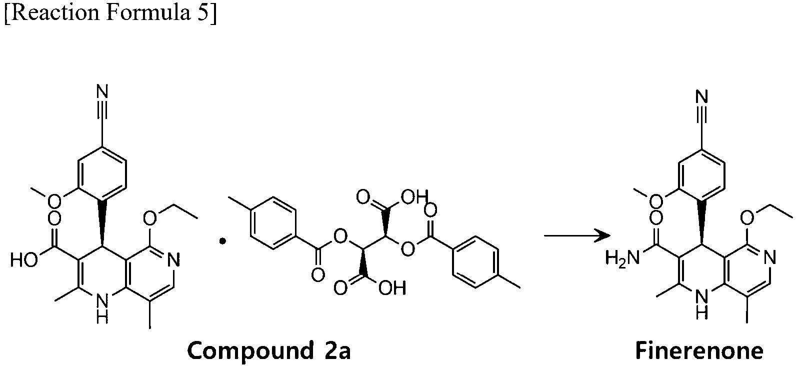

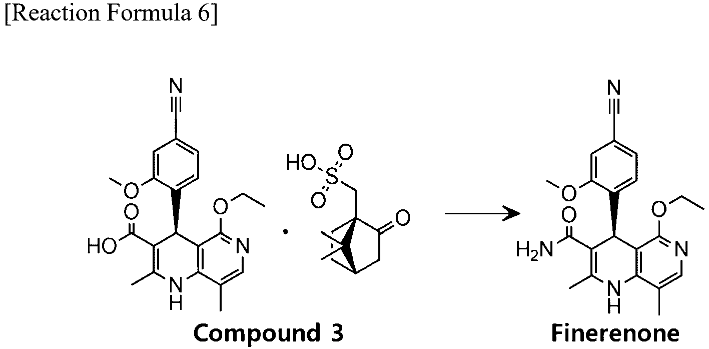

In the step of obtaining finerenone using compound 2a or 3 according to above reaction formulas 5 and 6, desalting and amide coupling may be performed in one process without performing a separated desalting process, thereby reducing the number of processes in the method for preparing finerenone.

In one embodiment, the method for preparing finerenone according to the present invention may be performed according to reaction formula 7 below.

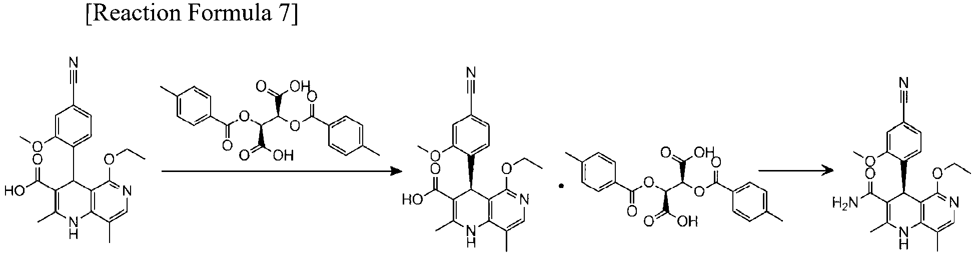

In one embodiment, the method for preparing finerenone according to the present invention may be performed according to reaction formula 8 below.

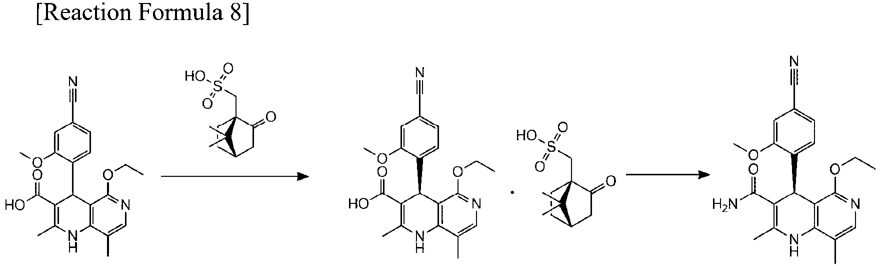

In one embodiment, the method for preparing finerenone may include:
a) a step of obtaining compound 2a represented by formula 2a or compound 3 represented by formula 3 by using compound 1 represented by formula 1;
b) a step of obtaining compound 1a by desalting compound 2a or 3;
c) a step of obtaining compound 2a or 3 by salting compound 1a; and
d) a step of obtaining finerenone by using compound 2a or 3 as a reactant of amide coupling.

The method for preparing finerenone may be substantially the same as described in the obtaining of compound 2a or 3; the obtaining of compound 1a and the obtaining of finerenone by using compound 2a or 3 as a reactant of amide coupling. Thus, any redundant detailed description will be omitted.

In one embodiment, compound 1, which is a racemic mixture, may be prepared by methods known in the art. For example, compound 1, which is a racemic mixture, may be prepared through substantially the same process as shown in the method described in Example 22A of Korean Registered Patent No. 10-1614164.

In one embodiment, compound 1, which is a racemic mixture, may be prepared through a step of obtaining compound 1b represented by formula 1b by desalting compound 2b represented by formula 2b; and a step of racemizing compound 1b.

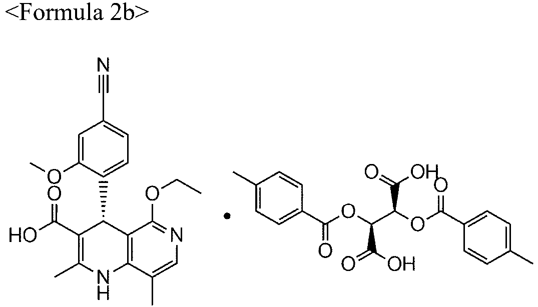

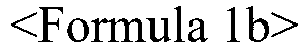

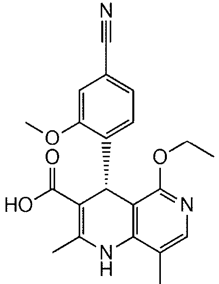

In this case, the method for preparing compound 1 may be performed according to reaction formula 9 below.

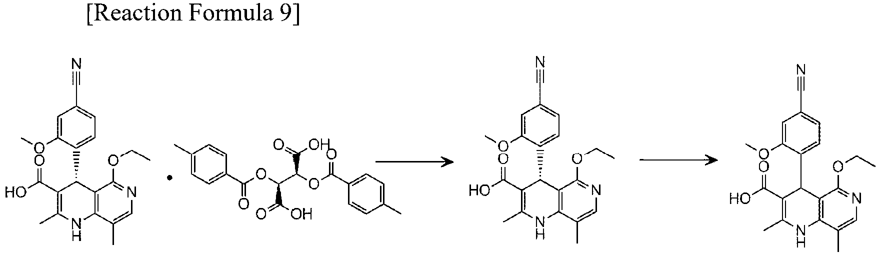

In one embodiment, compound 1, which is a racemic mixture, may be prepared through a step of obtaining compound 5 represented by formula 5 below by oxidizing compound 2b; and a step of obtaining compound 1 by reducing compound 5, in which a reaction is performed in a salt state without a separated desalting process, thereby obtaining compound 1 at a high yield.

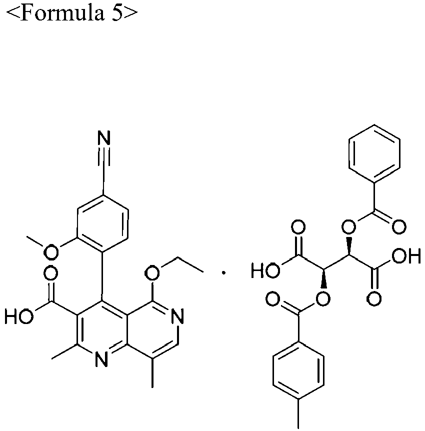

In this case, compound 1 may be prepared according to reaction formula 10 below.

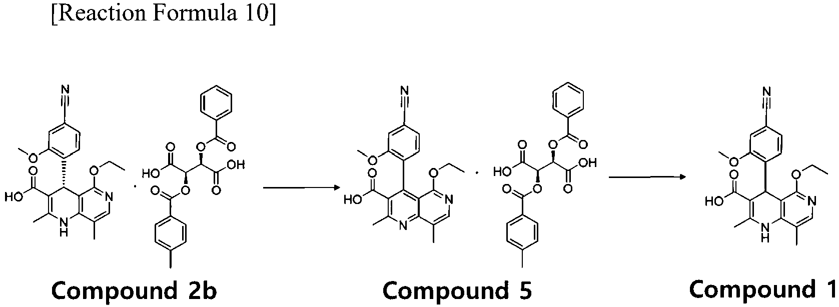

In one embodiment, above compound 2b may be obtained by reacting compound 1, which is prepared through substantially the same process as shown in the method described in Example 22A of Korean Registered Patent No. 10-1614164, with di-p-toluoyl-D-tartaric acid.

In one embodiment, the method for preparing finerenone of the present invention may further include:
after finishing at least one of the step of preparing compound 2a and the step of preparing finerenone from compound 2a,
a step of obtaining compound 1b by desalting compound 2b obtained in the step of preparing compound 2a;
a step of preparing compound 1 by racemizing compound 1b;
a step of preparing compound 2a or 3 using compound 1; and
a step of preparing finerenone represented by formula I from the obtained compound 2a or 3.

In one embodiment, the step of racemizing compound 1b may include:
a step of performing an oxidation reaction on compound 1b; and
a step of obtaining a racemic mixture in which compounds 1a and 1b are mixed by performing a reduction reaction on a product obtained by the oxidation reaction.

In one embodiment, the method for preparing finerenone of the present invention may further include:
after finishing at least one of the step of preparing compound 2a and the step of preparing finerenone from compound 2a,
a step of preparing compound 5 represented by formula 5 by oxidizing compound 2b obtained in the step of preparing compound 2a;
a step of preparing compound 1 represented by formula 1 by reducing compound 5;
a step of preparing compound 2a or 3 using compound 1; and
a step of preparing finerenone represented by formula I from the obtained compound 2a or 3.

In one embodiment, the step of preparing finerenone may include a step of obtaining finerenone by using compound 2a or 3 as a reactant of amide coupling.

The method for preparing finerenone may be substantially the same as described in the step of preparing finerenone by using compound 2a or 3. Thus, any redundant detailed description will be omitted.

In the method for preparing finerenone according to the present invention, compound 2b generated during the preparation process may not be immediately discarded, but may be utilized again in the preparation of compound 1 as a raw material capable of preparing compound 2a, thus providing an advantage of ultimately reducing the cost of preparing finerenone.

An intermediate compound for preparing finerenone for one object of the present invention may be compound 2a represented by formula 2a below or compound 2b represented by formula 2b below.

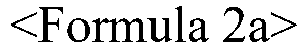

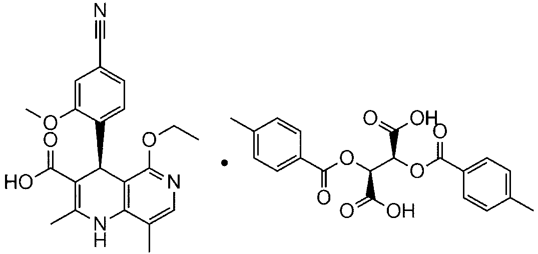

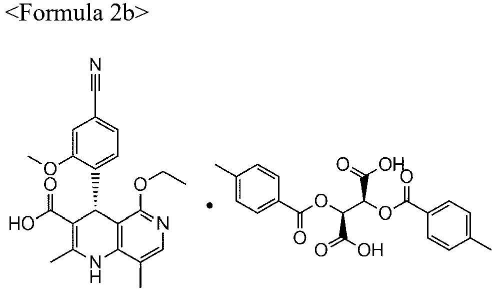

The present invention may provide a use of an intermediate compound, which is compound 2a or 2b, for preparation of finerenone and a use of an intermediate compound, which is compound 2a or 2b, for preparing finerenone.

The method for preparing compound 2a may be substantially the same as described in the preparing of compound 2a in the method for preparing finerenone. Thus, any redundant detailed description will be omitted. In other words, above compound 2a may be prepared according to above reaction formula 1.

The method for preparing compound 2b may be substantially the same as described in the preparing of compound 2a in the method for preparing finerenone. Thus, any redundant detailed description will be omitted. In other words, above compound 2b may be obtained by reacting compound 1 with di-p-toluoyl-D-tartaric acid, and then separating the same from the resulting product. Above compound 2b may be prepared according to above reaction formula 11.

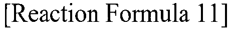

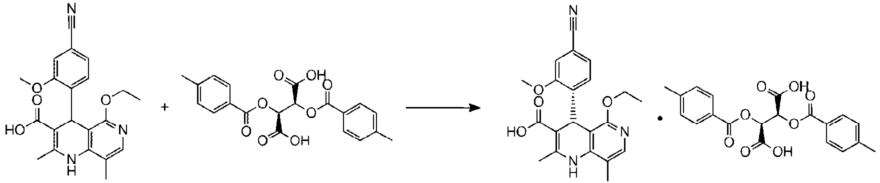

An intermediate compound for preparing finerenone for one object of the present invention may be compound 3 represented by formula 3 below.

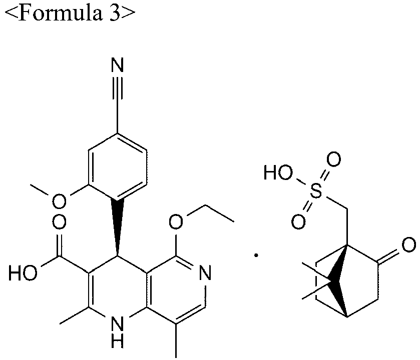

The present invention may provide a use of an intermediate compound, which is compound 3, for preparation of finerenone and a use of an intermediate compound, which is compound 3, for preparing finerenone.

The method for preparing compound 3 may be substantially the same as described in the preparing of compound 3 in the method for preparing finerenone. Thus, any redundant detailed description will be omitted. In other words, above compound 2b may be prepared according to above reaction formula 2.

In the present invention, each of compounds 2a and 3 may be a stable material, and may be easily obtained as a solid without a separate purification process. Compound 1, which is a racemic mixture, has low stability and thus has difficulty in handling, storage, and the like, but compound 2a or 3 may have excellent stability as an intermediate compound of finerenone and thus may be stably and easily used in a process for preparing finerenone. With respect to compound 1, which is a racemic mixture, a salt in a solid phase may not be obtained with compounds other than di-p-toluoyl-D-tartaric acid and (-)-camphor-10-sulfonic acid. In particular, even when tartaric acid or D-(+)-camphor acid is used as a material having a similar structure, a salt in a solid phase may not be obtained by reacting with compound 1, which is a racemic mixture.

The method for preparing compound 1 represented by formula 1 according to the present invention may include:
a step of obtaining compound 1b represented by formula 1b by desalting compound 2b; and
a step of obtaining compound 1 by racemizing compound 1b.

In one embodiment, the step of racemizing compound 1b may include:
a step of oxidizing compound 1b; and
a step of reducing a product obtained by an oxidation reaction, in which compound 1 may include compounds 1a and 1b.

In one embodiment, compound 4 represented by formula 4 below may be obtained in the step of oxidizing compound 1b. Compound 4 may be a racemic mixture having achirality, and compound 1 may be obtained by reducing compound 4.

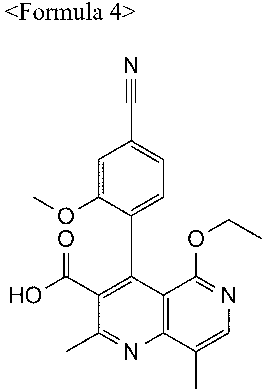

The method for preparing compound 1 represented by formula 1 according to the present invention may include:
a step of preparing compound 5 represented by formula 5 by oxidizing compound 2b; and
a step of preparing compound 1 represented by formula 1 by reducing compound 5.

In one embodiment, compound 5 represented by formula 5 below may be obtained in the step of oxidizing compound 2b.

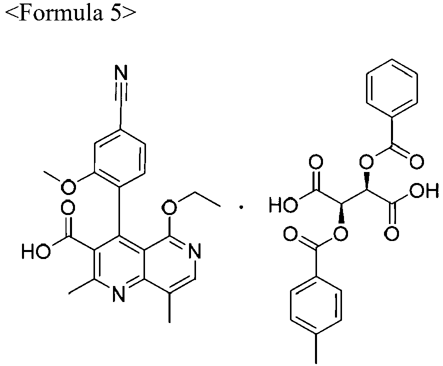

In one embodiment, the step of preparing compound 5 may be performed by reacting compound 2b and ceric ammonium nitrate (CAN) under a condition of an organic solvent. In this case, the organic solvent may be acetonitrile.

In one embodiment, the step of preparing compound 5 may include:
a step of reacting compound 2b and ceric ammonium nitrate in a presence of acetonitrile;
a step of extracting from ethyl acetate (EA) and water; and
a step of obtaining compound 5 as a solid by drying a concentrated residue under vacuum.

In one embodiment, in the mixing of compound 2b and ceric ammonium nitrate in a presence of acetonitrile to carry out a reaction, compound 2b and ceric ammonium nitrate may be mixed at an equivalent ratio of 1:1.5, and acetonitrile may be mixed in a volume of 40 times the weight of compound 2b.

In one embodiment, the step of reacting compound 2b and ceric ammonium nitrate in a presence of acetonitrile may be performed for one to three hours, and may be performed at room temperature.

In one embodiment, the obtaining of compound 1 may be performed by reacting compound 5, zinc powder, and ammonium formate under a condition of an organic solvent. In this case, the organic solvent may be THF.

In one embodiment, the step of obtaining compound 1 may include:
a step of reacting and refluxing compound 5, zinc powder and ammonium formate in a presence of THF;
a step of filtering zinc powder after cooling to 20°C;
a step of extracting by adding ethyl acetate (EA), water, and an aqueous NaCl solution;
a step of filtering and concentrating by adding MgSO₄ to an ethyl acetate (EA) layer; and
a step of refluxing a concentrated residue after adding acetonitrile to a concentrated residue;
a step of mixing after slowly cooling to 20°C; and
a step of obtaining compound 1 as a solid by washing with acetonitrile, filtering a solid, and drying under vacuum.

In one embodiment, in the step of reacting and refluxing compound 5, zinc powder and ammonium formate in a presence of THF, compound 5, zinc powder and ammonium formate may be mixed at an equivalent ratio of 1:15:15, and THF may be mixed in a volume of 25 times the weight of compound 5.

In one embodiment, the step of reacting and refluxing compound 5, zinc powder and ammonium formate in a presence of THF may be performed for one to two hours.
1) The present invention provides a method for preparing finerenone, the method comprising: a step of preparing compound 2a represented by formula 2a or compound 3 represented by formula 3 by using compound 1 represented by formula 1; and a step of preparing finerenone represented by formula I from compound 2a or 3: 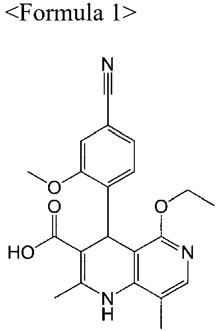 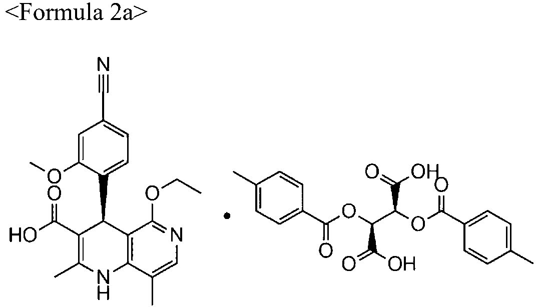 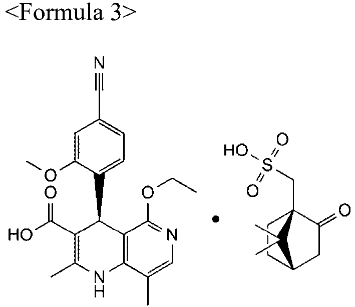 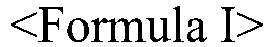 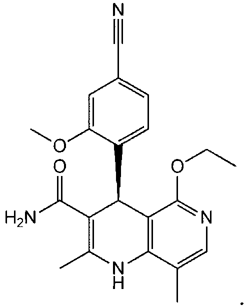
2) In 1), the step of preparing compound 2a or 3 may comprise a step of reacting compound 1 and di-p-toluoyl-D-tartaric acid or (-)-camphor-10-sulfonic acid.
3) In 2), compound 1 and di-p-toluoyl-D-tartaric acid or (-)-camphor-10-sulfonic acid may be reacted in a presence of acetonitrile.
4) In any one of 1) to 3), the step of preparing compound 2a or 3 may comprise a step of reacting compound 1 and di-p-toluoyl-D-tartaric acid or (-)-camphor-10-sulfonic acid at 15 to 90°C.
5) In any one of 1) to 4), the step of preparing of compound 2a may comprise a step of reacting compound 1 and di-p-toluoyl-D-tartaric acid in a presence of at least one organic solvent selected from the group consisting of methanol, ethanol, THF and acetonitrile.
6) In any one of 1) to 5), the step of preparing compound 3 may comprise reacting compound 1 and (-)-camphor-10-sulfonic acid in a presence of acetonitrile.
7) In 6), acetonitrile may be mixed in a volume of 10 times the weight of compound 1.
8) In any one of 1) to 7), the step of preparing finerenone may comprise a step of obtaining compound 1a represented by formula 1a by using compound 2a or 3: 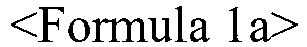 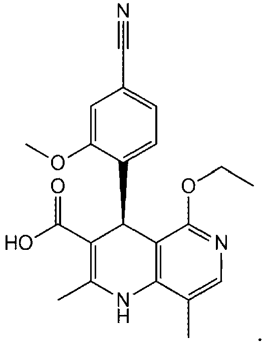
9) In 8), the step of obtaining compound 1a may be performed by adding disodium phosphate (Na₂HPO₄) under a condition of an aqueous alcohol solution to carry out a reaction.
10) In any one of 1) to 9), the step of preparing finerenone may comprise a step of obtaining finerenone by using compound 2a or 3 as a reactant of amide coupling.
11) In any one of 1) to 10), the step of obtaining finerenone by using compound 2a or 3 as a reactant of amide coupling may be performed by reacting 1-ethyl-3-(3-dimethylaminopropyl)carbodiimide (EDC), 4-dimethylaminopyridine (DMAP) and ammonium hydroxide (NH4OH) with compound 2a or 3 in ethyl acetate (EA).
12) In any one of 8) to 11), the step of preparing finerenone may further comprise a step of preparing compound 2a or 3 by salting the obtained compound 1a.
13) In 12), the step of preparing compound 2a may comprise a step of reacting compound 1a and di-p-toluoyl-D-tartaric acid by mixing compound 1a and di-p-toluoyl-D-tartaric acid at an equivalent ratio of 1:0.1 to 1:0.9.
14) In 12) or 13), the step of preparing compound 2a may comprise a step of reacting compound 1a and di-p-toluoyl-D-tartaric acid in a presence of at least one organic solvent selected from the group consisting of methanol and THF.
15) In any one of 8) to 14), the step of preparing finerenone may further comprise a step of obtaining compound 2a or 3 by using compound 1a after the step of obtaining compound 1a using compound 2a or 3, in which the step of obtaining compound 2a using compound 1a may comprise a step of reacting compound 1a and di-p-toluoyl-D-tartaric acid under a condition of an organic solvent, and compound 1a and di-p-toluoyl-D-tartaric acid may be reacted under a condition of an organic solvent comprising both methanol and THF, in which compound 1a and di-p-toluoyl-D-tartaric acid may be mixed at an equivalent ratio of 1:0.8, and methanol and THF may be mixed in volumes of 15 and 5 times the weight of compound 1a, respectively.
16) In any one of 1) to 15), the method for preparing finerenone may further comprise: a step of obtaining compound 5 represented by formula 5 by oxidizing compound 2b represented by formula 2b; and a step of obtaining compound 1 represented by formula 1 by reducing compound 5.
17) In any one of 1) to 16), the step of preparing finerenone may comprise a step of obtaining finerenone by using compound 2a or 3 as a reactant of amide coupling.
18) The present invention provides an intermediate compound for preparing finerenone, which is compound 2a represented by formula 2a below or compound 2b represented by formula 2b: 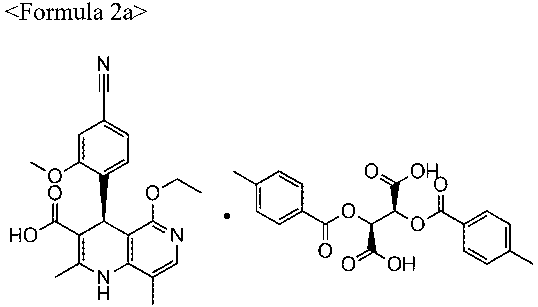 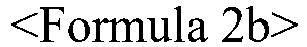 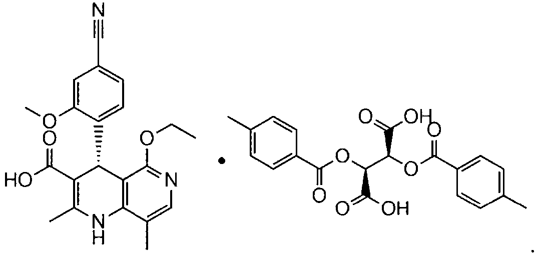
19) The present invention provides a method for preparing an intermediate compound for preparing finerenone, the method comprising: a step of preparing compound 2a represented by formula 2a or compound 2b represented by formula 2b by reacting compound 1 represented by formula 1 and di-p-toluoyl-D-tartaric acid: 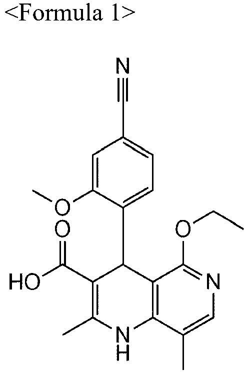 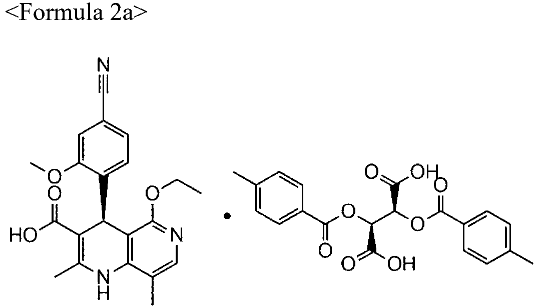 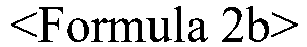 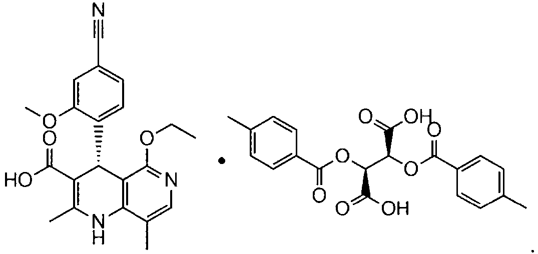
20) The present invention provides an intermediate compound for preparing finerenone, which is represented by formula 3 below: 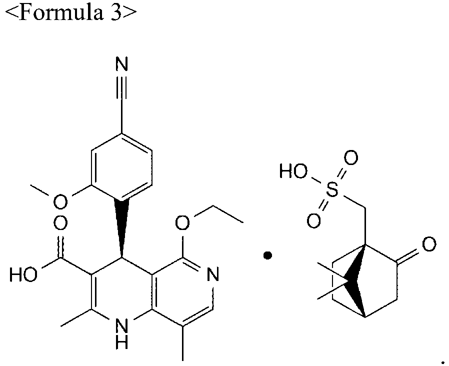
21) The present invention provides a method for preparing an intermediate compound for preparing finerenone, the method comprising: a step of preparing compound 3 represented by formula 3 by reacting compound 1 represented by formula 1 and (-)-camphor-10-sulfonic acid: 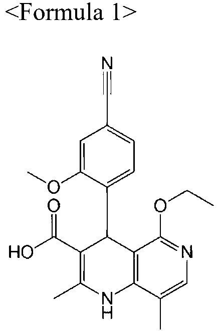 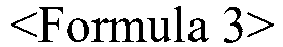 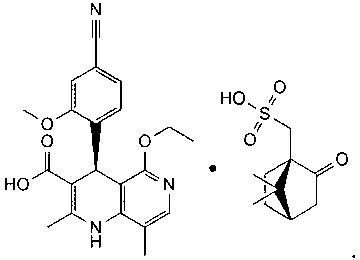
22) The present invention provides a method for preparing compound 1 represented by formula 1, the method comprising: a step of obtaining compound 5 represented by formula 5 below by oxidizing compound 2b represented by formula 2b; and a step of obtaining compound 1 represented by formula 1 by reducing compound 5: 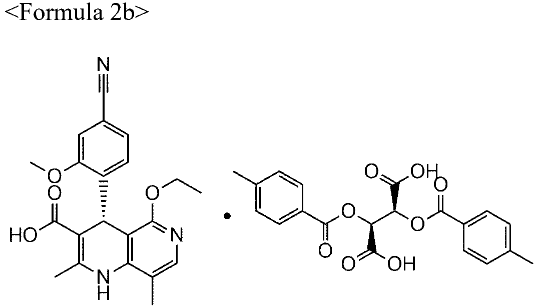 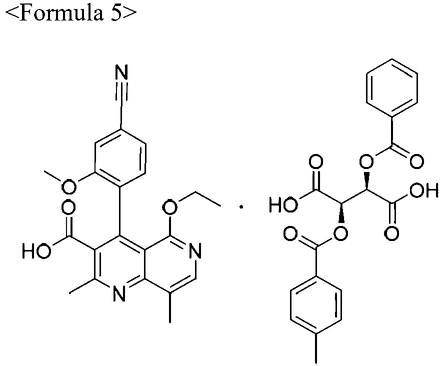 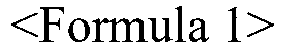 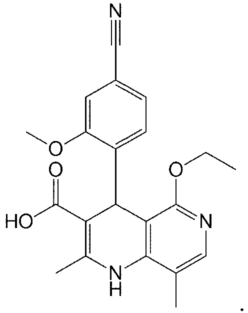
23) In 22), the step of obtaining compound 5 may be performed by reacting compound 2b and ceric ammonium nitrate (CAN) under a condition of an organic solvent. In this case, the organic solvent may be acetonitrile.
24) In 22) or 23), the step of obtaining compound 5 may comprise: a step of reacting compound 2b and ceric ammonium nitrate in a presence of acetonitrile; a step of extracting by adding ethyl acetate (EA) and water; and a step of obtaining compound 5 as a solid by drying a concentrated residue under vacuum.
25) In 24), in the reacting of compound 2b and ceric ammonium nitrate in a presence of acetonitrile, compound 2b and ceric ammonium nitrate may be mixed at an equivalent ratio of 1:1.5, and acetonitrile may be mixed in a volume of 40 times the weight of compound 2b. In addition, the reacting may be performed at room temperature for one to three hours; and/or
   in the step of extracting from ethyl acetate (EA) and water, ethyl acetate (EA) and water may be each mixed in a volume of 20 times the weight of compound 2b, and water may be further added to an ethyl acetate (EA) layer in a volume of 20 times the weight of compound 2b to carry out a re-extraction.
26) In any one of 22) to 25), the step of obtaining compound 1 may be performed by reacting compound 5, zinc powder, and ammonium formate under a condition of an organic solvent. In this case, the organic solvent may be THF.
27) In any one of 22) to 26), the step of obtaining compound 1 may comprise: a step of reacting and refluxing compound 5, zinc powder and ammonium formate in a presence of THF; a step of filtering zinc powder after cooling to 20°C; a step of extracting by adding ethyl acetate (EA), water, and an aqueous NaCl solution; a step of filtering and concentrating by adding MgSO₄ to an ethyl acetate (EA) layer; and a step of refluxing a concentrated residue after adding acetonitrile; a step of slowly cooling to 20°C and then mixing; and a step of obtaining compound 1 as a solid by washing with acetonitrile, filtering a solid, and then drying under vacuum.
28) In any one of 22) to 27), in the step of reacting and refluxing compound 5, zinc powder and ammonium formate in a presence of THF, compound 5, zinc powder and ammonium formate may be mixed at an equivalent ratio of 1:15:15, and THF may be added in a volume of 25 times the weight of compound 5. In addition, the step of refluxing may be performed for one to two hours;
   in the step of extracting by adding ethyl acetate (EA), water and an aqueous NaCl solution, ethyl acetate (EA), water and the aqueous NaCl solution may be mixed in volumes of 20, 20 and 10 times the weight of compound 5, and water may be further added to an ethyl acetate (EA) layer in a volume of 20 times the weight of compound 5 to carry out a re-extraction;
   in the step of filtering and concentrating by adding MgSO₄ to an ethyl acetate (EA) layer, MgSO₄ may be mixed in an amount of three times the weight of compound 5, further stirred for five minutes, and then filtered and concentrated;
   in the step of refluxing a concentrated residue after adding acetonitrile, acetonitrile may be mixed in a volume of twice the weight of compound 5 and may be further refluxed for 0.5 to one hour; and/or
   in the washing with acetonitrile to filter a solid and dry under vacuum, thereby obtaining compound 1 as a solid, acetonitrile may be used in a volume of twice the weight of compound 5.

### ADVANTAGEOUS EFFECTS

According to the method for preparing finerenone of the present invention as described above, each of compounds 2a and 3 can be a stable material, and can be easily obtained as a solid without a separate purification process. Compound 1, which is a racemic mixture, has low stability and thus has difficulty in handling, storage, and the like, but compound 2a or 3 can have excellent stability as an intermediate compound of finerenone. As such, compound 1 can be easily subjected to optical separation by using each of compounds 2a and 3, which are obtained as stable materials in a solid state from compound 1, which is a racemic mixture, as intermediate compounds.

In addition, although compound 1 is not optically separated from each of compounds 2a and 3, under certain conditions, amide coupling can be performed to prepare finerenone through a simple process while the optically separated material is removed at the same time.

Thus, according to the method for preparing finerenone of the present invention, it can be possible to prepare finerenone with a high preparation yield and a high optical purity, thereby improving the productivity of finerenone.

In addition, compound 2b can be used again to obtain compound 1, thereby ultimately reducing the preparation cost of finerenone.

### DETAILED DESCRIPTION OF THE EMBODIMENTS

Terms used in the present application are used only to describe a certain example and are not intended to limit the present invention. In the present application, terms such as "include," "have" or the like shall be intended to designate a presence of features, steps, operations, components, or combinations thereof described herein, and shall not be construed to exclude a possible presence or addition of one or more other features, steps, operations, components, or combinations thereof in advance. All the terms used herein including technical or scientific terms have the same meaning as commonly understood by those ordinary skilled in the art, to which the present invention pertains, unless defined otherwise.

### Preparation Example 1: Synthesis of 4-(4-cyano-2-methoxyphenyl)-5-ethoxy-2,8-dimethyl-1,4-dihydro-1,6-naphthyridin-3-carboxylic acid (Compound 1)

Compound 1, which is a racemic mixture, was prepared through substantially the same process as shown in the method described in Example 22A of Korean Registered Patent No. 10-1614164.

### Example 1 - Preparation of finerenone

### (Step 1) Preparation of di-p-toluoyl-D-tartrate salt of (S)-4-(4-cyano-2-methoxyphenyl)-5-ethoxy-2,8-dimethyl-1,4-dihydro-1,6-naphthyridin-3-carboxylic acid (Compound 2a)

Compound 1 (5 g, 1.0 eq) obtained according to Preparation Example 1 and di-p-toluoyl-D-tartaric acid (2.8 g, 0.55 eq) were added to acetonitrile (ACN, 175 ml) in a volume of 55 times the weight of compound 1. An internal temperature was raised to 80°C to carry out refluxing, and then the resulting mixture was stirred for one hour. Subsequently, the resulting mixture was cooled to 50°C for 30 minutes, aged at 50°C for one hour, cooled to 20 to 25°C for one hour, and then filtered. The solid obtained after washing with ACN (10 ml) in a volume of twice the weight of compound 1 initially used during filtration was dried under vacuum to obtain the title compound (optical purity of 93 to 94%, yield of 44.34%).

¹H NMR (500 MHz, DMSO-d₆) *δ* ppm 1.04 - 1.13(m, 6H), 2.14(s, 3H), 2.34 - 2.44(m, 10H), 3.41 - 3.47(m, 2H), 3.73(s, 3H), 3.97 - 4.10 (m, 2H), 4.37 (br s, 1H), 5.32(s, 1H), 5.82(s, 2H), 7.24 - 7.30(m, 2H), 7.31(s, 1H), 7.41(d, *J*=7.82 Hz, 4H), 7.57(s, 1H), 7.90(d, *J*=8.30 Hz, 4H), 8.16(s, 1H).

### (Step 2) Preparation of (S)-4-(4-cyano-2-methoxyphenyl)-5-ethoxy-2,8-dimethyl-1,4-dihydro-1,6-naphthyridin-3-carboxylic acid (Compound 1a)

After adding compound 2a (10 g, 1 eq) obtained in above step 1 into a 25% aqueous ethanol solution (100 ml) in a volume of 10 times the weight of compound 2a, 10% Na₂HPO₄ (150 to 250 ml) was added in a volume of 15 to 25 times the weight of compound 2a and stirred at 5 to 25°C for one to five hours, after which water (500 ml) was added to carry out filtering when the reaction was completed. During filtration, the resulting product was washed with a 25% aqueous ethanol solution (50 ml) in a volume of five times the weight of compound 2a, and the obtained solid was dried under vacuum to obtain the title compound (overall yield of 39.90% in steps 1 and 2).

### (Step 3) Preparation of finerenone

Compound 1a (10 g, 1.0 eq) obtained in above step 2, 1-ethyl-3-(3-dimethylaminopropyl)carbodiimide (EDCI, 7.58 g, 1.5 eq), 4-dimethylaminopyridine (DMAP, 483 mg, 1.5 eq), and 25% NH₄OH aqueous solution (21 ml, 5 eq) were added to tetrahydrofuran (THF) in a volume (100 ml) of 10 times the weight (10 g) of compound 1a to be used in step 3, and then a temperature was raised to 70 to 80°C to carry out refluxing. After one to two hours, the reaction was terminated when compound 1a remained at 1% or less, each of which was worked-up by using ethyl acetate (EA) (200 ml) and water (200 ml) in a volume of 20 times the weight of compound 1a, and then EA was concentrated. Ethanol (50 ml) was added in a volume of five times the weight of compound 1a, and then concentrated again. Ethanol (20 ml) was added to the concentrated solution in a volume of twice the weight of compound 1a, stirred at room temperature for 10 to 20 hours and then filtered. During filtration, a wash liquid used was ethanol (20 ml) in a volume of twice the weight of compound 1a, which was cooled to 0 to 10°C, and the obtained solid was dried under vacuum to obtain the title compound (purity of 97.07%, overall yield of 33.91% in steps 1 to 3).

### Example 2 - Preparation of finerenone

### (Step 1) Preparation of di-p-toluoyl-D-tartrate salt of (S)-4-(4-cyano-2-methoxyphenyl)-5-ethoxy-2,8-dimethyl-1,4-dihydro-1,6-naphthyridine-3-carboxylic acid (Compound 2a)

Compound 1 (10 g, 1.0 eq) obtained according to Preparation Example 1 and di-p-toluoyl-D-tartaric acid (2.8 g, 0.55 eq) were added to ethanol (2000 ml) in a volume of 200 times the weight of compound 1. The resulting mixture was stirred at an internal temperature of 15 to 25°C for 20 hours, and then filtered. During filtration, the resulting product was washed with ethanol (20 ml) in a volume of twice the weight of compound 1, and then the obtained solid was dried under vacuum to obtain the title compound (optical purity of 90.80%, yield of 43.66%).

### (Step 2) Preparation of (S)-4-(4-cyano-2-methoxyphenyl)-5-ethoxy-2,8-dimethyl-1,4-dihydro-1,6-naphthyridin-3-carboxylic acid (Compound 1a)

Compound 2a obtained in above step 1 was used to obtain the title compound through substantially the same process as shown in step 2 of Example 1 (overall yield of 39.29% in steps 1 and 2).

### (Step 3) Preparation of finerenone

Compound 1a obtained in above step 2 was used to obtain the title compound through substantially the same process as shown in step 3 of Example 1 (overall yield of 33.40% in steps 1 to 3).

### Example 3 - Preparation of finerenone

### (Step 1) Preparation of (-)-camphor-10-sulfonate salt of (S)-4-(4-cyano-2-methoxyphenyl)-5-ethoxy-2,8-dimethyl-1,4-dihydro-1,6-naphthyridin-3-carboxylic acid (Compound 3)

Compound 1 (10 g, 1.0 eq) obtained according to Preparation Example 1 and (-)-camphor-10-sulfonic acid (7.35 g, 1.2 eq) were added to ACN (5 to 10 ml) in a volume of five to 10 times the weight of compound 1. An internal temperature was raised to 80°C to carry out refluxing, and then the resulting mixture was stirred for one hour. Subsequently, the resulting product was cooled to 20 to 25°C for one hour, and then filtered. The solid obtained after washing with ACN (20 ml) in a volume of twice the weight of compound 1 during filtration was dried under vacuum to obtain the title compound (optical purity of 98.86%, yield of 21.96%).

¹H NMR (500 MHz, DMSO-d₆) *δ* ppm 0.74(s, 3H), 1.04(s, 3H), 1.17(t, J=7.08 Hz, 3H), 1.24 - 1.30(m, 2H), 1.81 - 1.87 (m, 2H), 1.93 (t, *J*=4.64 Hz, 1H), 2.20(s, 3H), 2.21 - 2.23 (m, 1H), 2.36 - 2.40 (m, 4H), 2.67 (t, *J*=10.99 Hz, 1H), 2.86(s, 1H), 4.06 - 4.13(m, 1H), 4.14 - 4.21(m, 1H), 5.32(s, 1H), 7.30 - 7.31(m, 1H), 7.36(s, 1H), 7.67(s, 1H), 8.53(br s, 1H).

### (Step 2) Preparation of (S)-4-(4-cyano-2-methoxyphenyl)-5-ethoxy-2,8-dimethyl-1,4-dihydro-1,6-naphthyridin-3-carboxylic acid (Compound 1a)

The title compound was obtained as a solid through substantially the same process as shown in step 2 of Example 1 except for using compound 3 obtained in above step 1 (overall yield of 20.11% in steps 1 and 2).

### (Step 3) Preparation of finerenone

Compound 1a obtained in above step 2 was used to obtain the title compound as a solid through substantially the same process as shown in step 3 of Example 1 (overall yield of 17.09% in steps 1 to 3).

### Example 4 - Preparation of finerenone

### (Step 1) Preparation of di-p-toluoyl-D-tartrate salt of (S)-4-(4-cyano-2-methoxyphenyl)-5-ethoxy-2,8-dimethyl-1,4-dihydro-1,6-naphthyridin-3-carboxylic acid (Compound 2a)

The title compound was obtained as a solid through substantially the same process as shown in step 1 of Example 1 (optical purity of 93 to 94%, yield of 44.34%).

### (Step 2) Preparation of finerenone

Compound 2a (10 g, 1.0 eq) obtained in above step 1, EDCI (3.76 g, 1.5 eq), DMAP (2.39 g, 1.5 eq), and 25% NH₄OH aqueous solution (10.4 to 31.2 ml, 5 to 15 eq) were added to EA (100 ml) in a volume of 10 times the weight of compound 2a to be used in step 2, and then a temperature was raised to 70 to 80°C to carry out refluxing. After one to two hours, the reaction was terminated when compound 1a remained at 1% or less, each of which was worked-up by using EA (200 ml) and water (200 ml) in a volume of 20 times the weight of compound 2a, and then EA was concentrated. Ethanol (50 ml) was added in a volume of five times the weight of compound 2a, and then concentrated again. Ethanol (20 ml) was added to the concentrated solution in a volume of twice the weight of compound 2a, and the resulting mixture was stirred at room temperature for 10 to 20 hours and then filtered. During filtration, a wash liquid used was ethanol (20 ml) in a volume of twice the weight of compound 2a, which was cooled to 10°C or less, and the obtained solid was dried under vacuum to obtain the title compound (overall yield of 39.6%, purity of 98.99% in steps 1 and 2).

### Comparative Example 1

With regard to compound 1 obtained according to Preparation Example 1, substantially the same process as the process described in steps 1 and 2 of Example 1 was performed except for using dibenzoyl-L-tartaric acid instead of di-p-toluoyl-D-tartaric acid. As a result, it was confirmed that compound 1b is obtained as a main product.

### Comparative Example 2

With regard to compound 1 obtained according to Preparation Example 1, substantially the same process as the process described in step 1 of Example 3 was performed except for using D-(+)-camphoric acid instead of (-)-camphor-10-sulfonic acid. As a result, it was confirmed that compound 3 is not prepared.

### Evaluation of properties - Temperature stability

An initial purity of compound 2a obtained according to an embodiment of the present invention was confirmed, and the purity was measured for Day 1, Day 3, Day 5, Week 1, Week 2, Week 3, and Week 4 while storing for up to Week 4 at 0°C, 25°C, and 50°C, respectively. As a result, it could be confirmed that compound 2a is not substantially changed compared to the initial purity until at least Week 4, even when stored at a temperature of 0°C, 25°C, and 50°C, respectively.

In addition, as a result of storing compound 3 obtained according to an embodiment of the present invention up to Week 4 at 0°C, 25°C, and 50°C in the same manner as compound 2a and comparing with the initial purity, it could be confirmed that there is substantially no change up to at least Week 4 compared to the initial purity.

### Example 5 - Preparation of compound 1

### (Step 1) Synthesis of compound 2b

Compound 2b was obtained through substantially the same process as shown in step 1 of Example 1.

### (Step 2) Synthesis of compound 1

### (Step 2a) Oxidation of compound 2b - Preparation of compound 5

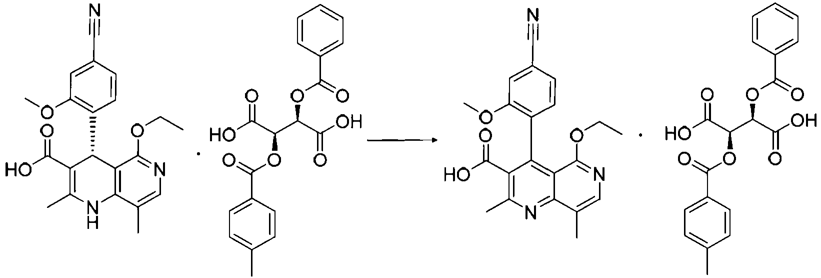

Ceric ammonium nitrate (CAN, 1.5 eq, 43.3 g) was added to the solid compound 2b (20 g, 1 eq) obtained in step 1 of Example 1 and ceric ammonium nitrate (CAN, 800 ml) was added thereto in a volume of 40 times the weight of compound 2b, and then stirred at room temperature for one to three hours. After the reaction was completed, EA (400 ml) and water (400 ml) were added in a volume of 20 times the weight of compound 2b, respectively, and an extraction was performed. Subsequently, water (400 ml) was added to an EA layer in a volume of 20 times the weight of compound 2b, and an extraction was performed again to completely concentrate the EA layer. Then, the concentrated residue was dried under vacuum (3 g, yield of 88.18% in step 2a). It was confirmed that the obtained product is obtained as compound 5.

### (Step 2b) Reduction of compound 5 - Preparation of compound 1

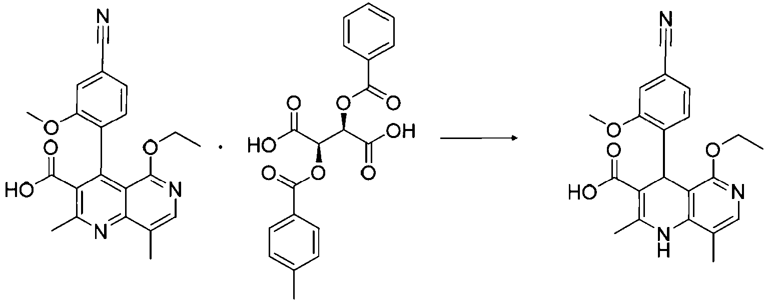

The solid (3 g, 1 eq) obtained in above step 2a, zinc powder (15 eq, 5.87 g), and ammonium formate (15 eq, 5.66 g) were added to THF (75 ml) in a volume of 25 times the weight of the solid obtained in above step 2a, heated to 64 to 65°C, and refluxed for one to two hours. Subsequently, the resulting mixture was cooled to 20°C, and then zinc powder was filtrated. Then, EA (60 ml), water (30 ml) and aqueous NaCl solution (30 ml) were added in volumes of 20, 10 and 10 times the weight of the solid obtained in above step 2a, respectively to perform an extraction, and then water (60 ml) was added to an EA layer in a volume of 20 times the weight of the solid obtained in above step 2a to perform an extraction again. In addition, MgSO₄ (9 g) was added to the EA layer in an amount of three times the weight of the solid obtained in above step 2a and stirred for five minutes, and then filtered and concentrated. Acetonitrile (ACN, 6 ml) was added to the concentrated residue in a volume of twice the weight of the solid obtained in above step 2a and refluxed at about 75 to 80°C for 0.5 to one hour. Subsequently, the resulting mixture was cooled to 20°C and then stirred for two to three hours. Then, after solid filtration (washing with acetonitrile in a volume (6 ml) of twice the weight of the solid obtained in step 2a), drying was performed under vacuum (1.62 g, yield of 88.93% in step 2b; overall yield of 78.25% in steps 1 and 2), and it was confirmed that the obtained product is obtained as compound 1, which is an achiral racemic mixture.

The present invention has been described with reference to preferred exemplary embodiments herein, but it will be understood by those skilled in the art that the present invention may be variously changed and modified without departing from the spirit and field of the present invention, as described in the following scope of patent claims.

## Claims

1. A method for preparing finerenone, the method comprising:
a step of preparing compound 2a represented by formula 2a or compound 3 represented by formula 3 by using compound 1 represented by formula 1; and
a step of preparing finerenone represented by formula I from compound 2a or 3:

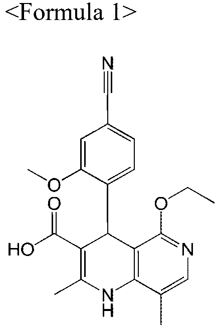

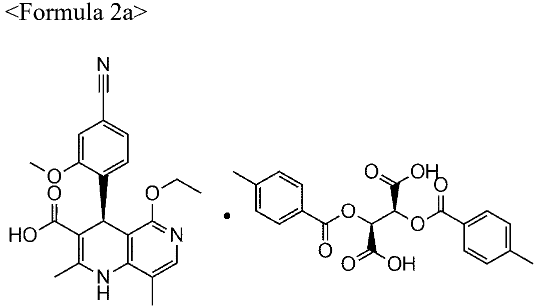

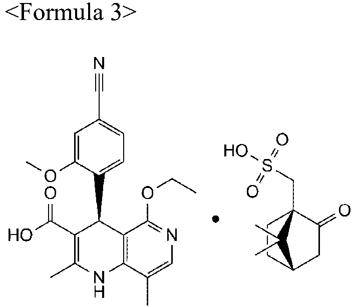

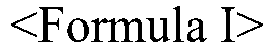

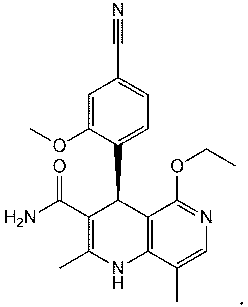

2. The method of claim 1, wherein the step of preparing compound 2a or 3 comprises a step of reacting compound 1 and di-p-toluoyl-D-tartaric acid or (-)-camphor-10-sulfonic acid.

3. The method of claim 2, wherein compound 1 and di-p-toluoyl-D-tartaric acid or (-)-camphor-10-sulfonic acid are reacted in a presence of acetonitrile.

4. The method of claim 2, wherein the step of preparing compound 2a or 3 comprises a step of reacting compound 1 and di-p-toluoyl-D-tartaric acid or (-)-camphor-10-sulfonic acid at 15 to 90°C.

5. The method of claim 1, wherein the step of preparing compound 2a comprises a step of reacting compound 1 and di-p-toluoyl-D-tartaric acid in a presence of at least one organic solvent selected from the group consisting of methanol, ethanol, THF and acetonitrile.

6. The method of claim 1, wherein the step of preparing compound 3 comprises a step of reacting compound 1 and (-)-camphor-10-sulfonic acid in a presence of acetonitrile.

7. The method of claim 6, wherein acetonitrile is mixed in a volume of 10 times a weight of compound 1.

8. The method of claim 1, wherein the step of preparing finerenone comprises a step of obtaining compound 1a represented by formula 1a by using compound 2a or 3:

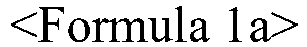

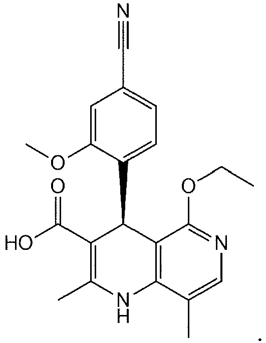

9. The method of claim 8, wherein the step of obtaining compound 1a has a reaction performed by adding disodium phosphate (Na₂HPO₄) under a condition of an aqueous alcohol solution.

10. The method of claim 1, wherein the step of preparing finerenone comprises a step of obtaining finerenone by using compound 2a or 3 as a reactant of amide coupling.

11. The method of claim 10, wherein the step of obtaining finerenone by using compound 2a or 3 as a reactant of amide coupling is performed by reacting 1-ethyl-3-(3-dimethylaminopropyl)carbodiimide (EDC), 4-dimethylaminopyridine (DMAP) and ammonium hydroxide (NH₄OH) with compound 2a or 3 in ethyl acetate (EA).

12. The method of claim 8, wherein the step of preparing finerenone further comprises a step of preparing compound 2a or 3 by salting the obtained compound 1a.

13. The method of claim 12, wherein the step of preparing compound 2a further comprises a step of reacting compound 1a and di-p-toluoyl-D-tartaric acid by mixing compound 1a and di-p-toluoyl-D-tartaric acid at an equivalent ratio of 1:0.1 to 1:0.9.

14. The method of claim 12, wherein the step of preparing compound 2a comprises a step of reacting compound 1a and di-p-toluoyl-D-tartaric acid in a presence of at least one organic solvent selected from the group consisting of methanol and THF.

15. The method of claim 1, wherein the method for preparing finerenone further comprises:
a step of obtaining compound 5 represented by formula 5 below by oxidizing compound 2b represented by formula 2b; and
a step of obtaining compound 1 represented by formula 1 by reducing compound 5:

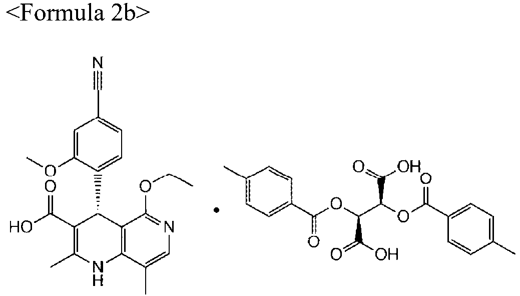

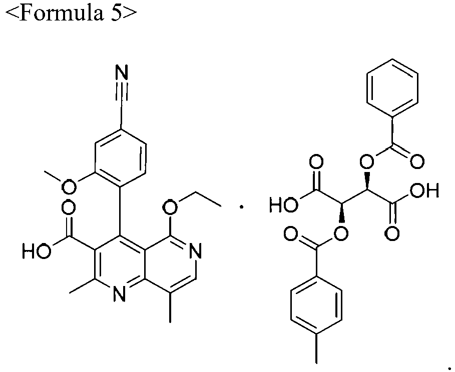

16. An intermediate compound for preparing finerenone, which is compound 2a represented by formula 2a below or compound 2b represented by formula 2b below:

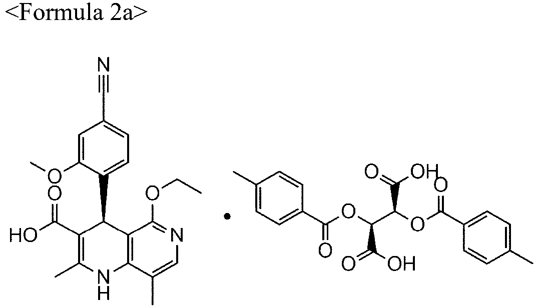

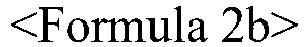

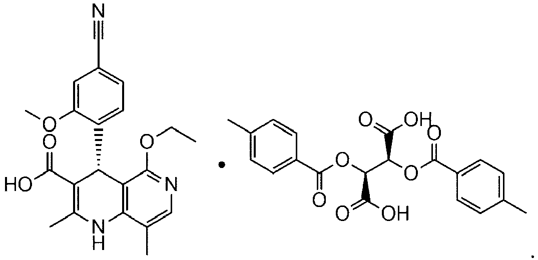

17. A method for preparing an intermediate compound for preparing finerenone, the method comprising:
a step of preparing compound 2a represented by formula 2a or compound 2b represented by formula 2b by reacting compound 1 represented by formula 1 and di-p-toluoyl-D-tartaric acid:

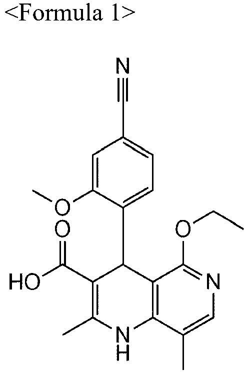

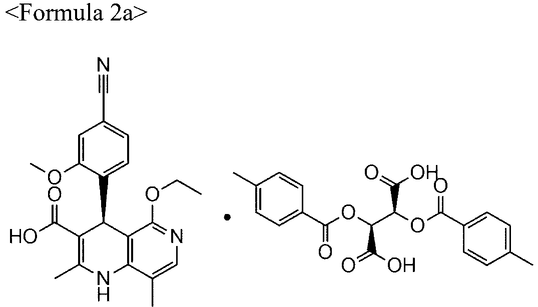

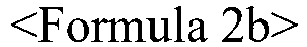

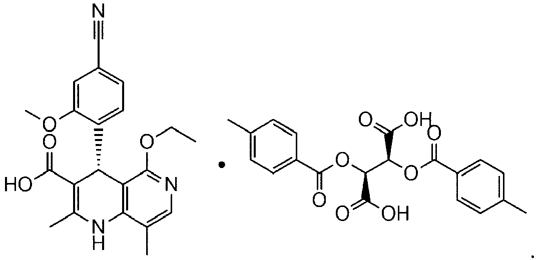

18. An intermediate compound for preparing finerenone, which is represented by formula 3 below:

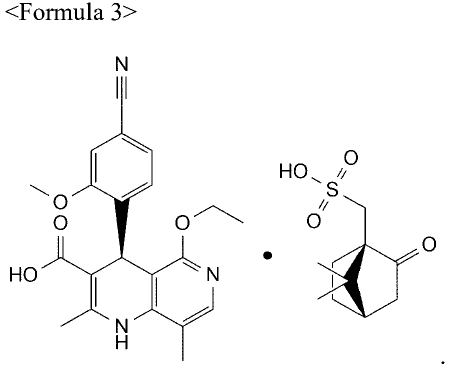

19. A method for preparing an intermediate compound for preparing finerenone, the method comprising:
a step of preparing compound 3 represented by formula 3 by reacting compound 1 represented by formula 1 and (-)-camphor-10-sulfonic acid:

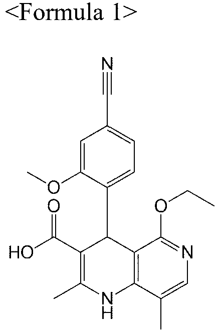

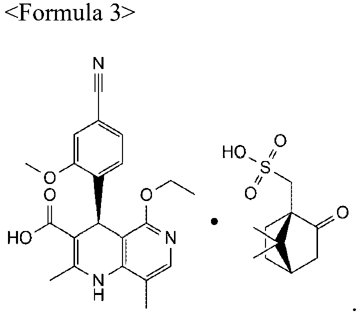

20. A method for preparing compound 1 represented by formula 1, the method comprising:
a step of obtaining compound 5 represented by formula 5 below by oxidizing compound 2b represented by formula 2b; and
a step of obtaining compound 1 represented by formula 1 by reducing compound 5:

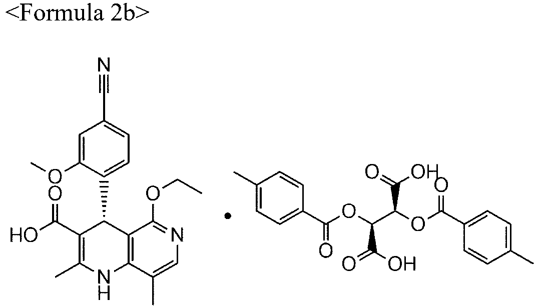

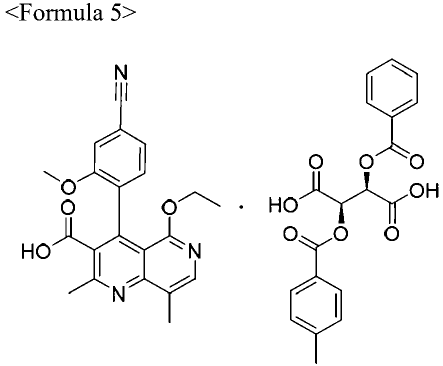

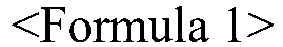

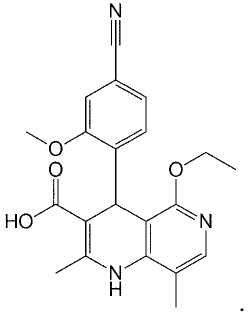

21. The method of claim 20, wherein the step of obtaining of compound 5 is performed by reacting compound 2b and ceric ammonium nitrate (CAN) in a presence of acetonitrile.

22. The method of claim 20, wherein the step of obtaining of compound 1 is performed by reacting compound 5, zinc powder, and ammonium formate in a presence of THF.
